# EUROPEAN PATENT APPLICATION

(11) **EP 3 566 577 A1**
(43) Date of publication of application: **13.11.2019**
(21) Application number: 18214204.2
(22) Date of filing: 19.12.2018
(51) Int. Cl.: A01N 25/08, A01N 25/12, A01N 25/34, A01N 59/16, A01N 59/20, E01C 13/08, B01J 20/16

(54) **AN ARTIFICIAL TURF INFILL MATERIAL FOR DISINFECTING ARTIFICIAL TURFS**

(30) Priority: 09.05.2018 US 201862668937 P
(71) Applicant: Polytex Sportbeläge Produktions-GmbH, 47929 Grefrath (DE); AstroTurf LLC, Dalton 30721 (US); Pintat, Benoit, 68340 Zellenberg (FR)
(72) Inventor: PINTAT, Benoît, 68340 Zellenberg (FR); SICK, Stephan, 47877 Willich-Neersen (DE); JANSEN, Bernd, 41334 Nettetal (DE); LOHR, Ivo, 47906 Kempen (DE); SHAHIN, Behnam Mokhber, 47798 Krefeld (DE); DIGERONIMO, David, Oceanside, CA 92057 (US); BROWN, Kristan Michael, Dalton, GA 30721 (US)
(74) Representative: Richardt Patentanwälte PartG mbB

(57) **Abstract**

The invention provides for a method of disinfecting an artificial turf structure (400A-C), comprising: applying to the artificial turf structure (400A-C) a mixture of microporous zeolite mineral and at least one of copper and silver.

## Description

### Field of the invention

The invention relates to artificial turf, in particular to artificial turf with infill, method of disinfecting artificial turf and also infill for artificial turf.

### Background and related art

Artificial turf or artificial grass is a surface that is made up of fibers which is used to replace grass. The structure of the artificial turf is designed such that the artificial turf has an appearance which resembles grass. Typically artificial turf is used as a surface for sports such as soccer, American football, rugby, tennis, golf, for playing fields, or exercise fields. Furthermore artificial turf is frequently used for landscaping applications. The contact of persons and animals with artificial as well as natural turf surfaces may result in clothing fibers, spit, dead skin cells, vomit, blood, or animal excrements such as animal urines being introduced into the artificial or natural lawn.

### Summary

The invention provides for a method of disinfecting artificial turf, method of forming an artificial turf structure, artificial turf and artificial turf infill in the independent claims. Embodiments are given in the dependent claims.

In some embodiments, a method of disinfecting an artificial turf structure is provided. The method comprises applying to the artificial turf structure a mixture of microporous zeolite mineral and at least one of copper and silver.

The present method may enable to sanitize the artificial turf surfaces and to remove or minimize the amount of bacteria e.g. bodily fluids. The introduction of organic matter like skin debris, sweat, blood, saliva and the like has hitherto, in the context of natural lawn, not been identified as a hygienical problem. This is because soil-based natural lawn comprises a complex microbiome that is typically able do degrade organic material of all kinds rapidly. Free nucleic acids, e.g. viral DNA or RNA, are rapidly degraded by extracellular nucleases secreted by various microorganisms, and pathogenic bacteria that may be contained in the spit of players or visitors are competed-out by non-pathogenic strains which are better adapted to the microclima of natural lawn. However, applicant has observed that the microclima in artificial turf is significantly different from that of natural lawn and that artificial turf may in some cases comprise a higher amount of organic material like some strains of bacteria or viruses that may have a pathogenic effect. Thus, reducing the amount of bacteria and/or viruses may be particularly advantageous in the context of artificial turf.

In a further beneficial aspect, adding microporous zeolite mineral (also referred to as zeolite mineral or zeolite) and at least one of copper and silver to artificial turf may be beneficial, because the antimicrobial substances are not an integral part of the artificial turf fibers and are therefore able to directly interact with any kinds of microorganisms, in particular bacteria. Hence, these antimicrobial substances do not have to migrate (slowly) out of the fibers before they can take effect.

In a further beneficial aspect, adding microporous zeolite mineral and at least one of copper and silver to artificial turf may be beneficial, because combining the copper and/or silver with the microporous zeolite material has been observed to provide for a synergistic effect: the zeolite itself may already have some antimicrobial and antiviral effect by adsorbing single-cell organism and virus particles to its porous surface structure. The metal ions work differently by being absorbed by the bacteria and interfering with their metabolism. It has been shown that the effect of combining a zeolite with at least one of copper or silver shows a stronger antimicrobial effect than would be expected based on a combination of the effects of the two substances alone. Without the intention to be bound by any theory, it is assumed that within the small cavities of the porous zeolite, the metal ions and the bacteria, fungi and viruses are brought into close contact with each other, thereby increasing the likelihood of interaction and the antimicrobial/antiviral effect of the metal ions.

The use of copper may be particularly advantageous as it is comparatively cheap and highly effective against bacteria, fungi as well as various viruses. For example, it has been observed that copper is able to significantly reduce the number of many well known pathogenic bacteria and viruses such as Acinetobacter baumannii (bacterium), Adenovirus (virus), Aspergillus niger (fungus), Candida albicans (fungus, yeast), Campylobacter jejuni (bacterium), Carbapenem-resistant Enterobacteriaceae (CRE), Clostridium difficile (bacterium), Coronavirus, Enterobacter aerogenes (bacterium), Escherichia coli O157:H7 (bacterium), Helicobacter pylori (bacterium), Influenza A (H1N1) (virus), Klebsiella pneumonia (bacterium), Legionella pneumophila (bacterium), Listeria monocytogenes (bacterium), Mycobacterium tuberculosis (bacterium), Norovirus or Norwalk-like virus, Poliovirus, Pseudomonas aeruginosa (bacterium), Salmonella enteritidis (bacterium), Staphylococcus aureus (MRSA, E-MRSA und MSSA), Tubercle bacillus (bacterium), Vancomycin-resistant enterococcus (VRE) (bacterium), and others.

Another advantage may be that odors such as urine odors may be limited as the zeolite mineral may adsorb animal excrements and/or urines. As the bacteria may be destroyed by the action of copper, this may further improve the limiting of unpleasant odors. The zeolite may for example adsorb a perfume to hide the residual odors.

The expression "to disinfect an object" as used herein is the act of applying one or more disinfectants to an object for reducing the number of living microorganisms and/or viruses that will be attached to the object within a future time interval starting from the application of the disinfectant to the object. Typically, the application of the one or more disinfectants also reduces the number of living microorganisms and/or viruses currently attached to said object.

Accordingly, a "disinfectant" is a substance that reduces the number of living microorganisms and/or viruses that will be attached to the object within a future time interval starting from the application of the disinfectant to the object. Typically, the disinfectant also reduces the number of living microorganisms and/or viruses currently attached to said object when the disinfectant is applied.

The term "microorganism" as used herein refers to single-cell organisms, in particular bacteria, archaea, fungi and protista.

For example, the future time interval can be a time interval of at last 10 weeks, or at least 10 month, or at least 2 years, starting from the time of disinfecting the object.

According to embodiments, the disinfectant is copper or silver or a combination thereof. According to some embodiments, also the zeolite is capable of reducing the number of living microorganisms and/or viruses now and in the future time interval and is thus a disinfectant.

The time interval during which the copper, the silver and the zeolite are capable of acting as a disinfectant may vary. For example, in some embodiments the zeolite may be clogged after several month or years with debris and the metal ions may be washed out after several month or years.

Depending on the type of disinfectant used, the reduction of the number of living microorganisms may be achieved via different biochemical pathways. For example, some disinfectants may destroy the cell membrane or cell wall of the microorganism at any physiological state or selectively in the growth state. Some other disinfectants may interfere with the metabolism such that the microorganisms cannot grow or cannot reproduce or lose their pathogenic properties. Thus, the number of microorganisms may be reduced even in case the disinfectant does not immediately kill all microorganisms. Thus, when a player spits on an artificial turf field, the disinfectants contained in the infill will prevent the microorganisms contained in the saliva of the player from growing and/or duplicating, thereby reducing the microbial load contained in the artificial turf.

The present method may save resources that would otherwise be required for repeatedly and manually disinfecting artificial turf surfaces and may thus limit or mitigate the human interventions needed for the disinfection process.

The mixture may include metal-loaded zeolites formed by mixing the microporous zeolite mineral and a metal (e.g. copper and/or silver) solution. The mixing may involve cation exchange of Cu2+ and/or Ag+. For example, the copper and/or silver may be put in minimum solution of water (preferably demineralized water to avoid exchanges with other cations) and to adsorb this aqueous solution by the zeolite at a rate of 50% of its weight. This may enable, after drying the mixture, that at least 5% of the mixture may be formed by copper in the porosities of the zeolite e.g. by cation exchange with a sodium ion Na +.

The term microporous zeolite mineral refers to porous zeolite mineral which is characterized by pore diameters that enable adsorption or absorption of copper and/or silver solution by the zeolite. The pore diameter may for example be of less than about 10nm (e.g. of about 1nm, 2nm, 3nm, 4nm, 6nm, 7nm to about 9nm).

According to one embodiment, the applying of the mixture comprises: applying the microporous zeolite mineral to the artificial turf structure; distributing or scattering the copper and/or silver on the applied microporous zeolite mineral (thereby obtaining the mixture); and exposing the mixture to water, thereby obtaining a copper and/or silver loaded zeolite. This embodiment enables a dry mixture of granules of zeolites and (e.g. 10% of) copper chloride. For obtaining a homogeneous mixture, the granule of the copper and zeolite may have similar sizes or have substantially the same size. This embodiment enables to first mix dry elements containing zeolite with copper (e.g. in hydroxide form) and/or silver before exposing the dry mixture to water. The exposition to water may complete the mixture process as the copper and/or silver (being wet) may be loaded into (or adsorbed or absorbed by) the zeolite. The humidity generated by water (e.g. from the rain) may enable to form metal solution. The mixing process is performed on the artificial turf surfaces. This embodiment may enable an efficient and cost effective method for disinfecting the artificial turf structure.

According to one embodiment, the method further comprises redistributing the copper and/or silver on the applied microporous zeolite mineral after a predefined time period and exposing the resulting mixture of zeolite mineral and copper and/or silver to water (for obtaining again a copper and/or silver loaded zeolite). The zeolite mineral may have an excellent cation exchange capacity. The urine of animals which are loaded with cationic elements, in particular the sodium, may cause the release of copper when the urine is in contact with the zeolite mineral. For example, during the predefined time period the artificial turf surface may receive animal urines. This may cause cation exchange between the copper and the sodium ions of the urines, leading to a progressive release of the copper from the pores of the zeolite mineral. After the predefined time period, the zeolite mineral has lost at least part of the copper from the pores. Thus repeating the method may ensure that the disinfection capability of the artificial turf is maintained overtime.

For example, the zeolite enables an exchange capacity over a long time period. By receiving animal's urines and/or following the leaching caused by the rains or caused by watering, the copper may progressively be released. This zeolite may however remain loaded with other mineral or metallic cations resulting from the rains or watering, such as sodium, potassium, calcium, magnesium, and/or ammonium urine. It may therefore be advantageous and easy to add or mix granules of copper hydroxide with this zeolite to redo an exchange cycle with these conventional components of urine. The contact or application of the copper to the zeolite may trigger a release of the sodium. For example, as the zeolite may regenerate with sodium, this may enable to release a sodium ion in favor of the capture of a calcium ion or a copper ion.

According to one embodiment, the zeolite mineral has a size in the range [0.42 mm, 1.39 mm], or [0.59 mm, 1.39 mm], or [0.84 mm, 1.68 mm], or [0.18mm, 0.25mm]. This embodiment provides the optimal size ranges for the dry mixture of the previous embodiment.

According to one embodiment, the mixture comprises a copper and/or silver loaded zeolite that is obtained before being applied to the artificial turf structure. This embodiment enables to first produce the mixture before being applied or scattered to the artificial turf surface. This embodiment may enable an optimal control of the production of the mixture as described hereinafter.

According to one embodiment, the obtaining of the mixture comprises: adding or mixing the copper (e.g. the copper may be in the form of copper-chloride, cuprous oxide, copper oxychloride or other chemical declensions of copper) and/or the silver to water, thereby obtaining a metal solution; mixing the metal solution with the microporous zeolite mineral, resulting in an aqueous mixture; drying the aqueous mixture. This embodiment may enable an optimal and controlled preparation of the mixture for obtaining an improved disinfection capability. The copper and/or silver is provided in an amount of at least 5% to 10% of the metal solution e.g. in form of water-soluble oxychloride. In one example, the copper and/or silver form 30% to 40% of the metal solution. The water is provided in an amount of at least 60% of the metal solution. For example, the copper is dissolved in water in an amount of 5 to 10% of the solution. This amount of the copper and/or silver enables a better adsorption of the metal in the zeolite pores because the solution may not be heavy.

According to one embodiment, the mixing is performed such that the microporous zeolite mineral adsorbs at least 40 to 50% of the metal solution. For example, the copper may progressively dissolve under effect of humidity generated by rains or watering of the turf surface. Since the zeolite is a cation exchanger, it may absorb or adsorb de metal solution (e.g. like a sponge). The copper may then enter pores of zeolite mineral. The zeolite mineral may however lose over time the copper by cation exchange with sodium that results from animal urines e.g. an exchange between ion copper and ion sodium may occur.

For example, the solution may comprise 5% to 10% of copper and 90% to 95% of water. The zeolite mineral may adsorb 50% of the solution such that the amount of copper in the 50% adsorbed solution is part of the mixture of copper and zeolite. For example, after the drying, the copper may form at least 5% of the mixture.

In one example, the zeolite mineral may adsorb 50% by weight of the microporous zeolite mineral. For example, the copper that is mixed with the zeolite may comprise 54 % of metal copper.

According to one embodiment, the metal solution and the microporous zeolite mineral are exposed to a predefined pressure. This embodiment may increase the quantity of copper and/or silver adsorbed or absorbed by the zeolite mineral.

According to one embodiment, the method further comprises introducing the metal solution together with the microporous zeolite mineral in an autoclave at the predefined pressure (e.g. for a predefined time period). For example, the preparation of the mixture (involving aqueous mixture water and copper and/or silver) may be done under pressure in the autoclave to increase the adsorbed and absorbed amount of the zeolite. This embodiment may enable better control on the pressure at which the metal solution can be obtained is performed. For example, the copper e.g. in oxychloride form, may dissolve very quickly in water solution. Pressurization for a few minutes (3 to 5 min) may therefore be sufficient such that the copper penetrate the pores of the zeolite mineral.

According to one embodiment, the water is a demineralized water. Using demineralized water may prevent exchange with other cations.

According to one embodiment, the water is obtained from one of the rain and a sprinkler system. This may further simplify the present method and further reduce the human intervention in the mixing process.

According to one embodiment, the mixture is applied to the artificial turf structure in amount of 25 g/m² to 2500 g/m². For example, the amount is determined based on the level of disinfection needed or on the level of bacteria expected on the artificial turf surface.

According to one embodiment, the porosity of the zeolite mineral is about 10 % to about 40%, preferably from about 10% to about 35%.

According to one embodiment, the specific surface area of the microporous zeolite mineral is between 25 and 40 m²/g.

According to one embodiment, the microporous zeolite mineral has a selected grain size smaller than 1.5mm and a porosity of about 15% to about 20%.

According to one embodiment, the grain size distribution of said microporous zeolite mineral is as follows: 70-90% of the grains have a size in the range of about 0.4mm to about 1.5mm and about 10% to about 30% of the grains have a grain size smaller than about 0.4mm.

In some embodiments, a method for manufacturing or forming an artificial turf (e.g. an artificial turf with a disinfection capability) is provided. The method comprises: providing an artificial turf structure; applying the microporous zeolite mineral to the artificial turf structure; distributing of the copper and/or silver on the applied microporous zeolite mineral; exposing the mixture to water. For example, the provided artificial turf surface may comprise an artificial turf carpet. The artificial turf carpet comprises a backing and also artificial grass fibers. The artificial grass fibers are tufted into the backing and are secured to the backing. The artificial turf fibers form a pile. The artificial turf carpet is resting on a ground or surface. Between and distributed between the artificial grass fibers and within the pile is the mixture.

According to one embodiment, the zeolite mineral has a size in the range [0.42 mm, 1.39 mm], or [0.59 mm, 1.39 mm], or [0.84 mm, 1.68 mm], or [0.18mm, 0.25mm].

In some embodiments, an artificial turf infill material comprises a copper and/or silver loaded zeolite is provided. The copper and/or silver loaded zeolite may be obtained as described with the previous embodiment.

According to one embodiment, the zeolite has a selected gain size smaller than 1.5 mm and a porosity between 15% and 20%.

According to one embodiment, the zeolite has a grain size distribution as follows: 70% to 90% of the grains have a size in the range [0.4 mm, 1.5 mm] and 10% to 30% of the grains have a size smaller than 0.4 mm.

According to one embodiment, 0.6% of the zeolite at most is not retainable on a 100 mesh screen.

According to one embodiment, the microporous zeolite mineral having a hardness smaller than 3 or smaller than 4 on the Mohs scale.

According to one embodiment, the moisture level in the mineral is smaller than 6% or smaller than 20%. In this case, the usage of dry zeolite mineral may thus be advantageous as it may help adsorption of the metal solution.

In some embodiments, an artificial turf is provided. The artificial turf comprises an artificial turf carpet with a pile and artificial turf infill, wherein the artificial turf carpet comprises a backing; wherein the artificial turf carpet further comprises artificial grass fibers, wherein the artificial grass fibers are tufted into the backing, wherein the artificial grass fibers form the pile, wherein the artificial grass fibers are secured to the backing, wherein the artificial turf infill comprises a copper and/or silver loaded zeolite.

According to one embodiment, the artificial turf further comprises a sprinkler system.

In one example, the use of copper and/or silver loaded zeolite for disinfecting an artificial turf structure is provided.

The microporous zeolite mineral of the above embodiments may for example be obtained as described with the following examples.

In one example, a method for forming an artificial turf infill material (or the microporous zeolite mineral of the above embodiments) is provided. The method comprises: providing a zeolite ore; and selecting from the zeolite ore a microporous zeolite mineral using a selection criterion on specific surface area of the mineral, thereby providing the artificial turf infill material. The selected microporous zeolite mineral may be used for the mixture described above. For example, the selection may be performed by sorting by size the grains of the mineral such that the grains having a size higher than a predetermined maximum grain size are rejected or filtered out and the grains having a size bellow the maximum grain size form the microporous zeolite mineral.

The artificial turf may be used for a number of applications such as sporting venues, landscape applications and green roofs on buildings. However, on playing fields, for example, the outside surfaces of the artificial turf are subject to the constraints of bad weather and temperature variations that lead to problems in maintaining these outside surfaces in the conditions optimal for use. In order to solve such a problem, the present method provides a purposive selection of the zeolite mineral. This may enable to address specific purposes of the use of the artificial turf infill, wherein such purposes can be controlled via the specific surface area. One example purpose is to enable the use of artificial turfs within the framework of bad weather and high temperatures.

The determination of the specific surface area of the mineral is particularly relevant for monitoring industrial processing of the zeolite minerals. The specific surface area constitutes an important criterion that enables the determination of the quality of a zeolite mineral since the nature of the specific surface area enables a decisive characteristic for the overall usage of zeolite. In particular the usage of the selected microporous zeolite mineral as an artificial turf infill may have an impact on how realistically the artificial turf performs.

Artificial turf infill is a material that covers the bottom portion of the artificial turf fibers. The use of artificial turf infill of the present disclosure may further have a number of advantages. For example, artificial turf infill may help the artificial turf fibers stand up straight. Artificial turf infill may also absorb impact from walking or running and provide an experience similar to being on real turf. The artificial turf infill may also help to keep the artificial turf carpet flat and in place by weighting it down.

The selection of the microporous zeolite mineral may for example comprise crushing the zeolite ore for obtaining groups of zeolite materials. The specific surface area of each group of the groups may be determined or measured. Based on the measured specific surface area groups that fulfill the selection criterion may be selected to form the microporous zeolite mineral. The specific surface area may for example be measured by adsorption using the Brunauer, Emmett, and Teller (BET) technique. This may have the advantage of measuring the surface of fine structures and deep texture on the particles.

The selection criterion refers to rules (e.g. classification rules) on the basis of which it may be decided whether the specific surface area of zeolite material is to be selected for forming the microporous zeolite mineral of the present method.

According to one embodiment, the selection criterion comprises: the specific surface area is smaller than a predetermined maximum specific surface area. Without putting an upper limit on the specific surface area, the infill material may comprise an inhomogeneous combination of the zeolite mineral. This embodiment may further be advantageous as it may be simple to implement in particular where mineral production involves repeated processes.

According to one embodiment, the method further comprises determining the maximum specific surface area of the mineral as the surface specific area that enables the water in the mineral to release, under an ambient temperature, at a predefined minimum rate. This embodiment may enable a progressive release of the water by the microporous mineral and thus may avoid rapid evaporation of the water after watering the surface. This may allow a lower temperature to be maintained at the level of the field surface compared to the ambient temperature. For example, the controlled release of water causes progressive cooling under evaporation. Thus the amount of watering usually necessary to refresh a field surface may be reduced.

The present selected grain structure of the mineral enables the formation of bound water surrounding mineral surfaces and maintained by weak force of van der Waals force. This renders the release or desorption of the water easier in particular under ambient temperature (e.g. the solar energy is enough to desorb the water). While the bound water is releasing the water inside the grain may be transferred under ambient temperature to the grain's surface which is then transformed to water vapor. This may enable a progressive release of water over a predefined time period and may enable the cooling of the surface of the artificial turf.

According to one embodiment, the selecting comprises: determining a zeolite grain size corresponding to the maximum surface specific area; providing a grinding unit; reducing the zeolite ore into smaller zeolite fractions; setting parameters of the grinding unit in accordance with the determined grain size; repeatedly grinding and screening the zeolite fractions in the grinding unit for selecting the microporous zeolite mineral. The grain size may refer to the diameter (e.g. maximum diameter) of individual grains.

The repeatedly grinding and screening may comprise repeating the grinding and the screening. In one example, the step of screening may comprise multiple screening. The multiple screenings may be of the same or different type screenings. For example, a screening of the multiple screening may comprise an inclined screening and another screening of the multiple screening may comprise a horizontal screening. Performing multiple screening may enable an optimal dedusting of the resulting microporous mineral.

According to one embodiment, the zeolite fractions have a maximum size between 1.2 mm and 1.9 mm.

According to one embodiment, the reducing of the zeolite ore comprises crushing the zeolite ore in a primary crusher, wherein the grinding unit comprises a secondary crusher and a screening unit, wherein the parameters comprise at least one of: the number of decks of the screening unit, the number of times the screening is to be repeated in the screening step; the exciting force causing the vibration of the screening unit; inclined and/or horizontal screening; reduction ratio of the primary and secondary crushers. A high number of parameters enable an optimal control of the grinding unit for providing an efficient production and selection of the microporous zeolite mineral.

According to one embodiment, the method further comprises drying the smaller zeolite fractions in a dryer before the grinding. Dust generated during mineral production activities provides a pathway for the accumulation of contaminants in the surrounding environment. This embodiment may have the advantage of reducing the amount of dust in the resulting microporous zeolite mineral.

According to one embodiment, the method further comprises shaping the microporous zeolite mineral in a predefined shape, wherein the maximum specific surface area is determined based on the solar reflectivity of the zeolite material having the predefined shape. The solar reflectivity depends on the shape of the reflecting object. By controlling the shape of the microporous zeolite mineral, the rate of the water release may be more efficiently and uniformly controlled. This is because the range of the temperature under which the artificial turf is, may be controlled by the solar reflectivity.

For example, the minimum rate may be determined for an ambient temperature between min1 and max1 regardless of the shape of the microporous zeolite mineral. If the artificial turf is to be implemented in a region having an ambient temperature between min2 and max2, the shape of the microporous zeolite mineral may be chosen such that the temperature at the surface of the turf is between min1 and max1.

According to one embodiment, the artificial turf infill is the microporous zeolite mineral. The microporous zeolite mineral is the only infill material. This may provide safe and environmental friendly artificial turfs.

According to one embodiment, the porosity of the zeolite ore is between 15% to 20%, wherein the maximum specific surface area is between 20 m²/g and 35 m²/g. In a preferred embodiment the maximum specific surface area is between 15 m²/g and 25 m²/g. In a very preferred embodiment the maximum specific surface area is between 19 m²/g and 21 m²/g. For example, the selected specific surface area may be 20 m²/g the microporous zeolite mineral having a porosity of 20%.

According to one embodiment, the ambient temperature is between 40° C and 60° C or below 100° C. "Ambient temperature" refers to a temperature of the air that surrounds the artificial turf under circumstances without any special heating and cooling. For example, the minimum rate may be determined using the usage time of the artificial turf. For a playing field, the minimum rate may be determined based on the game duration time e.g. such that the water progressively releases during the entire game.

According to one embodiment, the method further comprises: determining the maximum specific surface area such that in the presence of humidity, the mineral absorbs the humidity.

The humidity absorption refers to the moisture buffering capacity of the microporous zeolite mineral. This embodiment may prevent, for example, in the cold season the appearance of frost which renders the surfaces hard and slippery and thus dangerous to use.

According to one embodiment, the microporous zeolite mineral has a grain size between 0.5 mm and 1.2 mm or between 0.9 mm and 1.2 mm or between 0.2 mm and 1 mm. The selected size may have the further advantage of protecting the users of the artificial turf by reducing the risk of skin injury when the users are in contact the infill material. This may also prevent a slippery surface of the artificial turf.

According to one embodiment, 0.6% of the mineral is not retainable on a 100 mesh screen. This may provide another means (e.g. in combination with the drying) for further controlling the amount of dust in the resulting microporous zeolite mineral.

According to one embodiment, the microporous zeolite mineral has a hardness between 3 and 4 on the Mohs scale. This may provide a soft and resilient playing surface. This may reduce the risk of injuries (e.g. skin abrasion). Another advantage may be that the present infill material by reducing the wear effect of synthetic turf fibers caused by the friction between the zeolite mineral and the fibers.

For example, the amount of arsenic in the microporous zeolite mineral is below 4 mg per kg of the mineral. This may provide a healthy material.

In some embodiments, a method for controlling the temperature on an artificial turf is provided. The method comprises providing a microporous zeolite mineral having a selected specific surface area of the mineral; and using the microporous zeolite mineral as an infill material of the artificial turf.

According to one embodiment, the microporous zeolite mineral has a color with a predefined brightness, wherein the specific surface area of the mineral being selected based on the predefined brightness. The color may for example be white and the brightness may be equal to 85. This provides an additional parameter for an optimal control of the temperature at the surface of the artificial turf. The determination of the specific surface area may be modulated or combined with the brightness by balancing between the two parameters values in order to obtain the minimum rate.

In some embodiments, an artificial turf is provided. The artificial turf comprises an artificial turf carpet with a pile and artificial turf infill, wherein the artificial turf carpet comprises a backing; wherein the artificial turf carpet further comprises artificial grass fibers, wherein the artificial grass fibers are tufted into the backing, wherein the artificial grass fibers form the pile, wherein the artificial grass fibers are secured to the backing, wherein the artificial turf infill comprises a microporous zeolite mineral having a selected specific surface area of the mineral.

According to one embodiment, the artificial turf further comprises a sprinkler system. The use of a sprinkler system with the artificial turf may be beneficial because it may be used to automatically wet the artificial turf infill. For example this may be a convenient means of watering the artificial turf during a time period that is defined based on the minimum release rate of the water from the microporous zeolite mineral. For example, the selected microporous mineral may have a specific surface area which enables the water to progressively release during the half time period of a football game. In this case, the sprinkler system may be configured to water the artificial turf during the half-time break of the game.

In some embodiments, an artificial turf infill material is provided. The artificial turf infill material comprises a microporous zeolite mineral having a selected gain size smaller than 1.5 mm and a porosity between 15% and 20%.

According to one embodiment, the microporous zeolite mineral has a grain size distribution as follows: 70% to 90% of the grains have a size in the range [0.4 mm, 1.5 mm] and 10% to 30% of the grains have a size smaller than 0.4 mm. In another example, the microporous zeolite mineral has a grain size distribution as follows: 70% to 90% (e.g. 88%) of the grains of the microporous zeolite mineral have a size in the range [0.42 mm, 1.41 mm] (14-40 mesh) and 10% to 30% (e.g. 12%) of the grains of microporous zeolite mineral have a size smaller than 0.42 mm. The selected sizes may enable to obtain the selected specific surface area. In one example, the grain size of the microporous zeolite mineral may be within the range 14-100 mesh.

The microporous zeolite mineral has a grain size that enables the mixing of the zeolite mineral with the copper and/or silver in order to obtain a metal-loaded zeolite in accordance with the present disclosure. In one example, the grain size of the microporous zeolite mineral may be within the range 14-100 mesh. In another example, the grain size of the microporous zeolite mineral may vary from 4 to 500 mesh or between 12 and 20 mesh.

According to one embodiment, 0.6% of the mineral at most may not be retainable on a 100 mesh screen.

According to one embodiment, the microporous zeolite mineral has a hardness between 3 and 4 on the Mohs scale.

The infill material of any of the preceding claims 22-25, wherein the moisture level in the mineral is smaller than 6%

It is understood that one or more of the aforementioned embodiments of the invention may be combined as long as the combined embodiments are not mutually exclusive.

### Brief description of the drawings

In the following embodiments of the invention are explained in greater detail, by way of example only, making reference to the drawings in which:
- Fig. 1: is a flowchart of a method for disinfecting an artificial turf structure;
- Fig. 2: is a flowchart of an example method for applying metal-loaded zeolite on the artificial turf structure;
- Fig. 3: is a flowchart of a method for forming an artificial turf infill material;
- Fig. 4: is a flowchart of an example method for selecting a microporous zeolite mineral from the zeolite ore;
- Fig. 5: is a flowchart of another example method for selecting a microporous zeolite mineral from the zeolite ore;
- Fig. 6A: illustrates an example of an artificial turf;
- Fig. 6B: illustrates a further example of an artificial turf;
- Fig. 6C: illustrates a further example of an artificial turf; and
- Fig. 7: illustrates an example of an artificial turf which incorporates a sprinkler system.

### Detailed Description

Like numbered elements in these figures are either equivalent elements or perform the same function. Elements which have been discussed previously will not necessarily be discussed in later figures if the function is equivalent.

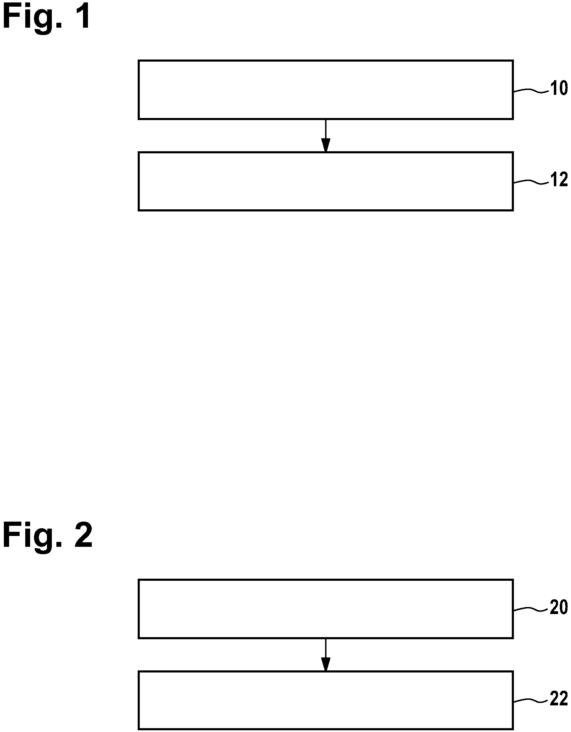

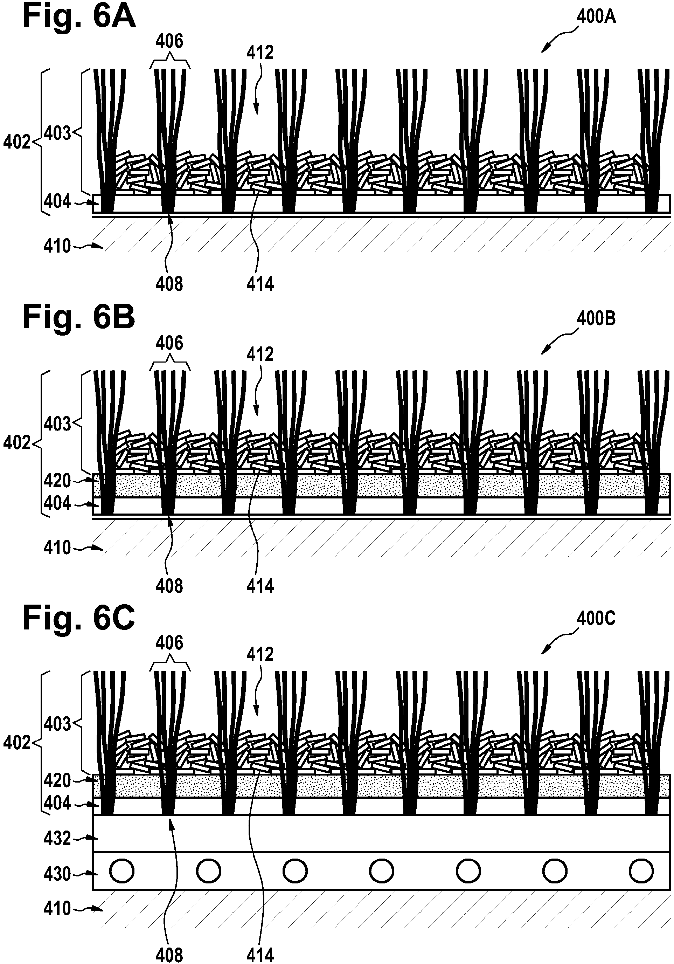

**Figure 1** is a flowchart of a method of disinfecting an artificial turf. In step 10, an artificial turf structure (such as artificial turf structure described with reference to Figures 6A-6C) is provided. In step 12, a mixture of microporous zeolite mineral and at least one of copper and silver may be applied to the artificial turf structure. The application of the mixture may for example be performed by first preparing the mixture and then applying or scattering it on the artificial turf structure.

The method of Figure 1 enables, for example, a dry mixture of granules of zeolites and copper chloride. The dry elements containing zeolite and copper (e.g. in hydroxide form) and/or silver are mixed by applying both of them on the turf surface. After application of the dry elements of zeolite and copper/or silver, the elements are exposed to water e.g. of rains. This exposition to water may complete the mixture process as the copper and/or silver (being wet) may be loaded into (or adsorbed or absorbed by) the zeolite. The humidity generated by water (e.g. from the rains) may enable to form metal solution with the copper and/or silver.

For example, during a predefined time period the artificial turf surface may receive animal urines. This may cause cation exchange between the copper and the sodium ions of the urines, leading to a progressive release of the copper from the pores of the zeolite mineral. After the predefined time period, the zeolite mineral may lose at least part of the copper from the pores. After the time period, at least part of the method may be repeated to ensure that the disinfection capability of the artificial turf is maintained over time. For that, the zeolite mineral (that received the urines) may be left on the artificial turf and the copper and/or silver may be applied again to the existing zeolite mineral. The exposition of the applied elements to water may enable the copper and/or silver to enter the pores of the zeolite again as described above.

**Figure 2** is a flowchart of a method for applying the mixture of Figure 1 on the artificial turf structure. In step 20, the copper and/or the silver may be added to or mixed with water, thereby obtaining a metal solution. In step 22, the metal solution is mixed with the microporous zeolite mineral, resulting in an aqueous mixture (e.g. in which the zeolite has adsorbed or absorbed at least part of the metal solution). In step 23, the aqueous mixture is dried.

As described with reference to Figure 1, after the predefined time period, the zeolite mineral (resulting from the method of Figure 2) may lose at least part of the copper from the pores. After the time period, the zeolite mineral (that received the urines) may be left on the artificial turf and the copper and/or silver to the existing zeolite mineral may be applied again. The exposition to water of the applied elements may enable the copper and/or silver to enter the pores of the zeolite again.

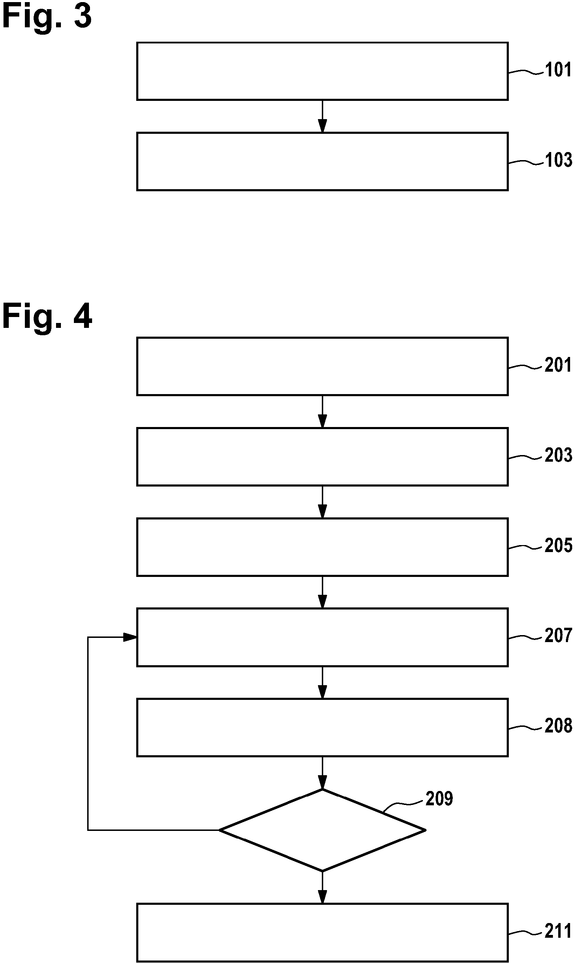

The microporous zeolite mineral of Figures 1 and 2 may for example be obtained or selected as described with reference to Figures 3 and 4.

**Figure 3** is a flowchart of a method for forming an artificial turf infill material. The infill material may be included in artificial turfs as described with reference to Figures 4A-5.

In step 101, a zeolite ore may be provided. The zeolite ore is a naturally occurring solid material. The zeolite ore has a porosity between of 15% and 20%. The term "porosity" refers to the volume fraction of void space in a porous article. The zeolite phase of the zeolite ore may comprise one or more of the group consisting of clinoptilolite, mordenite, or other naturally occurring zeolite minerals.

The zeolite ore may be provided for example as follows. A zeolite deposit is stripped of overburden and stockpiled for use in subsequent mine reclamation. The resulting exposed ore body is drilled to depths between 12 and 14 feet. The drill holes are loaded with an explosive charge that degenerates upon use, leaving no residue in the zeolite ore. From the mine pit, the zeolite ore is hauled by dump truck to the crude ore stockpile at a processing mill.

In step 103, a microporous zeolite mineral may be selected from the zeolite ore. The selection may be performed using a selection criterion involving the specific surface area of the mineral. The selection criterion may refer to one or more rules on the specific surface area.

For example, the selection of step 103 comprises a selective recovering or obtaining of the microporous zeolite mineral having a predefined specific surface area from the zeolite ore. The specific surface area constitutes an important criterion that is involved in the determination of the quality of a zeolite mineral since the nature of the specific surface area enables a decisive characteristic for the overall usage of zeolite in numerous technical components and products. For example, a specific surface area which is too high may render the release of water under ambient temperature very slow or inexistent.

In one example, the selection criterion requires that the specific surface area is smaller than a predetermined maximum specific surface area. The maximum specific surface area of the mineral may for example be determined as the surface specific area that enables the water in the mineral to release, under an ambient temperature, at a predefined minimum rate. For example, for a specific surface area equal or higher than 40 m²/g the water may only release under temperatures which are higher than the maximum ambient temperatures. Those high temperatures require the use of an oven. The present method may be advantageous as the maximum surface specific area that is selected enables the water to release under ambient temperatures e.g. between 40 and 60 C. The selected specific surface area may for example be 20 m²/g for a porosity of 15% to 20%.

In another example, the maximum specific surface area is chosen such that at most 0.6% of the mineral is not retainable on a 100 mesh screen e.g. 94% of the mineral has a grain size higher than 0.15 mm. This may have the advantage of reducing the amount of dust in addition to enabling a progressive release of the water for an optimal cooling of the artificial turf. Reducing the amount of dust may be beneficial for improving the safety of the product as regards the protection of the respiratory system of users of the artificial turf.

The selected microporous zeolite mineral may be used as the artificial infill material. In on example, the artificial infill material may consist of the selected microporous zeolite mineral. In another example, the artificial infill material may comprise the selected microporous zeolite mineral in addition to other infill materials.

**Figure 4** is a flowchart of an example method for selecting a microporous zeolite mineral from the zeolite ore (e.g. the zeolite ore provided in step 101) using a grinding unit. The grinding unit is configured for performing the grinding and screening of zeolite materials. The grinding unit may have parameters for controlling its function. The parameters may for example comprise the reduction ratio of the grinding unit, the number of times the screening is to be repeated in the screening step; the exciting force causing the vibration of the screening unit; inclined and/or horizontal screening.

In step 201, a zeolite grain size that corresponds to the maximum surface specific area may be determined. The grain size of the microporous zeolite mineral is determined such that the resulting specific surface area of the mineral is smaller than the maximum specific surface area.

Naturally, the specific surface area of the microporous zeolite mineral varies with its structure. For example, the finer the mineral is, the larger the specific surface area is (i.e. the smaller the grain size is, the larger the specific surface area is).

For example, the specific surface area of the microporous zeolite mineral may not exceed a minimum specific surface area. The minimum specific surface area may be the smallest possible specific surface area. In this case, the determined grain size may be the lower limit of a range of sizes, wherein the upper limit of the range may be determined using the minimum specific surface area. The microporous zeolite mineral may for example have a grain size between 0.5 mm and 1.2 mm or between 0.9 mm and 1.2 mm, for a maximum surface specific surface area of 21m²/g (e.g. the selected specific surface area may be 20 m²/g).

In step 203, the zeolite ore may be reduced into smaller zeolite fractions. Figure 3 shows an example method for reducing the zeolite ore into smaller fractions. The zeolite fractions may for example have a maximum size of 5/8 inch. In order to obtain that maximum size for the fractions, the reducing of the zeolite ore may comprise in addition to crushing the zeolite ore, a sieving or screening step, wherein in the screening step the crushed zeolite ores are screened with series of sieves. For example, the series of sieves may comprise sieves having sieve sizes ranging from about a minus 14 mesh (1.41 mm) to about a plus 40 mesh (0.42 mm).

In step 205, parameters of the grinding unit may be set in accordance with the determined grain size of step 201. For example, the reduction ratio of the grinding unit may be set such that the grinding unit may provide or output from the zeolite fractions grains having as a maximum size the determined grain size.

In one example, before performing the grinding step 207, the zeolite fractions resulting from step 203 may be dried in a dryer. This may have the advantage of reducing the amount of dust in the resulting microporous zeolite mineral.

In step 207, the zeolite fractions may be grind in the grinding unit. The term "grinding" encompasses processes like cutting, chopping, crushing, milling, pulverizing, and the like.

After grinding the zeolite fractions, the resulting zeolite material may be screened in step 208, resulting in groups of zeolite grains, wherein each group has a respective minimum grain size. The screening may for example be performed using series of sieves having sieve sizes ranging from about a minus 14 mesh (1.41 mm) to about a plus 40 mesh (0.42 mm). The screening may be a vibratory-type screening.

In one example, up to six or more different fractions can be separated in one screening process. This may for example be done using multiple sieve decks positioned on top of each other in a classification range such as a range of 0.1 mm to 1.5 mm.

The maximum grain size of each group of the groups may be compared with the determined grain size of step 201. In case (inquiry 209) the minimum grain size of a group of the groups is higher than the determined grain size, step 208 or steps 207-208 may be repeated. Otherwise, the group may be selected and stored in step 211 as part of the selected microporous zeolite mineral.

For example, the method may end if the selected microporous zeolite mineral reaches a predefined amount or if the input ore is completed.

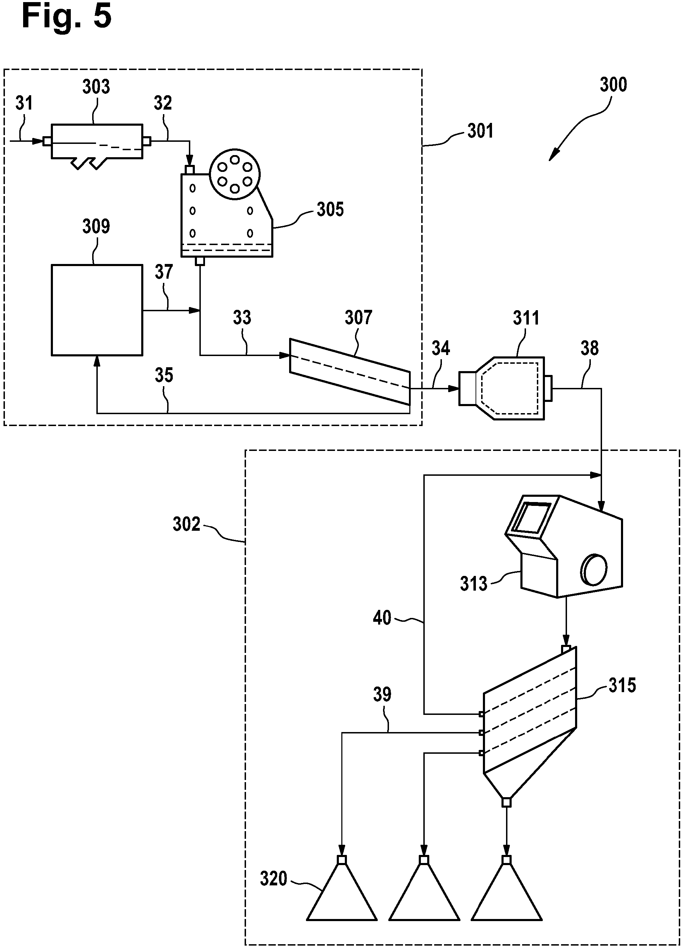

**Figure 5** illustrates the process of selecting a microporous zeolite mineral from a zeolite ore (e.g. zeolite ore of step 101) in accordance with another example of the present disclosure. Figure 3 shows a crushing unit 301 and a grinding unit 302, wherein the zeolite ore is first processed at the crushing unit 301 and the resulting material is input to the grinding unit 302 for further processing.

Before processing the zeolite ore in the crushing unit 301, the zeolite ore may for example be obtained as follows. A zeolite deposit is stripped of overburden and stockpiled for use in subsequent mine reclamation. The resulting exposed ore body is drilled to depths between 12 and 14 feet. The drill holes are loaded with an explosive charge that degenerates upon use, leaving no residue in the zeolite ore. From the mine pit, the zeolite ore is hauled by dump truck to the crude ore stockpile at a processing mill.

The zeolite ore is fed in step 31 through a grizzly 303 with 16"x16" opening, the output ore of the grizzly 303 travels in step 32 via a first conveyer into a jaw crusher 305 where the output ore of the grizzly 303 is reduced to a 4 inch size resulting in 4inch ore. The 4 inch ore travels in step 33 via a second conveyor to a double deck Nordberg screen 307 with a 5/8 inch screen on the top deck. The resulting output of the double deck Nordberg screen 307 is a minus 5/8 inch material and plus 5/8 inch material.

The minus 5/8 inch material travels in step 34 to a third conveyor toward the grinding unit 302 via a dryer 311. The plus 5/8 inch material travels back in step 35 via a fourth conveyor to a cone crusher 309 which reduces the plus 5/8 inch material to at least ½ inch material. The ½ inch material then returns in step 37 to the Nordberg screen 307 via the second conveyor.

From the third conveyor, the zeolite material output of the Nordberg screen 307 travels in step 34 to a propane fueled rotary kiln dryer 311 where it is heated at 250°C, reducing moisture to 5%, and fed in step 38 to the grinding unit 302.

The zeolite material is conveyed in step 38 from the dryer 311 via a fifth conveyor to an impact crusher 313 and five-decked Midwestern screens 315. From the screens 315 the zeolite is sized and conveyed in step 39 to a sixth conveyor for packaging e.g. in super sacks 320 ready to ship or the zeolite is returned in step 40 via a seventh conveyor to the impact crusher 313 and which is returned to the Midwestern Screens 315. At the Midwestern screens 315, the products are sized according to customer specifications and either sent to finished product handling.
In the finished product handling process: a. the material is either sent to bulk storage silos for direct truck loading or b. The material is sent to packaging silos where it is packaged in customer specified bags and palletized, wrapped, and stored in warehouse for truck pick up.

The following table gives example properties of the selected microporous zeolite mineral of the present method.

| **Parameter** | **Values** |
|---|---|
| Granulometry | 14x40 mesh (0.42 - 1.39 mm) |
| Particle size distribution | |
| 14 mesh (1.39 mm) | 0.9 % |
| 20 mesh (0.84 mm) | 39.0 % |
| 30 mesh (0.59 mm) | 27.0 % |
| 40 mesh (0.42 mm) | 21.0 % |
| 100 mesh (0.15 mm) | 10.0 % |
| < 100 mesh | 0.6 % |
| color / Brighthness | White / 85 |
| Hardness | 3 |
| Porosity | 15 - 20% |
| Arsenic total | < 4 mg/kg sec |
| Level of humidity | ≤ 6 % |

The values of the parameters, particle size or grain size, color, hardness, arsenic total and level of humidity, listed in the table are central values. However, each parameter of these parameters may have a value in the range defined by the central value, ± 10%, ± 5% or ± 3% of the central value. These values may for example enable to obtain a specific surface area of 20m²/g.

**Figure 6A** shows an example of an artificial turf (or artificial turf structure) 400A. The artificial turf 400A comprises an artificial turf carpet 402. The artificial turf carpet comprises a backing 404 and also artificial grass fibers 406. The artificial grass fibers 406 are tufted into the backing 404 and are secured 408 to the backing 404. The artificial turf fibers 406 form a pile 403. The artificial turf carpet 402 is resting on a ground 410 or surface. Between and distributed between the artificial grass fibers 406 and within the pile 403 is an artificial turf infill 412. The infill artificial turf infill 412 is shown as having a cylindrical shape; however it may have other shapes. For example, the shape of the microporous zeolite mineral may be a spherical shape. In this example the artificial turf infill 412 is made from at least the selected microporous zeolite mineral 414. In on example, the artificial infill material may consist of the selected microporous zeolite mineral. In another example, the artificial infill material may comprise the selected microporous zeolite mineral in addition to other infill materials. In another example, the artificial infill material may comprise the mixture of the zeolite mineral with at least one of the copper and the silver.

**Figure 6B** shows a further example of an artificial turf (or artificial turf structure) 400B. The artificial turf 400B is similar to the artificial turf 400A shown in Figure 4A except there is additionally a sand layer 420 between the artificial turf infill 412 and the backing 404. The use of the sand layer 420 may be advantageous because it may help to hold the artificial turf carpet 402 in place. It may also have the technical benefit that the sand layer 430 works in conjunction with the artificial turf infill 412 to regulate the amount of water on the surface of the artificial turf 400B. For example if it rains or if water is sprayed onto the surface of the artificial turf 400B the composite infill components 414 may rapidly absorb and saturate with water. The sand layer 420 may then aid in draining away excess water and preventing it from standing on the surface of the artificial turf 400B.

**Figure 6C** shows a further example of an artificial turf (or artificial turf structure) 400C. The artificial turf 400C is similar to the artificial turf 400B shown in Figure 4B with the addition of several additional layers. Directly underneath the backing 404 is an elastic layer 432. The elastic layer 432 may for example be a mat or other material such as sand and elastomeric granulate or a mixture thereof that readily absorbs shock. The elastic layer 432 is optional. The backing 404 and/or the elastic layer 432 may have holes or may be porous so that water that is standing on the artificial turf 400C can be drained away. The elastic layer 432 is directly sitting on a drainage system 430. The drainage system 430 may comprise granulate material, drainage tiles, drainage pipes or other system for rapidly draining water off the surface of the artificial turf 400C. The artificial turf depicted in Figure 4C may have superior qualities when water is used to cool or improve sliding properties. Water that initially goes on the surface may readily be absorbed by the composite infill components 414 that make up the artificial turf infill 412. When they have filled with water excess water may then go into and possibly be stored in the sand layer 420. When the sand layer 420 is saturated it may drain through the backing 404 and/or the elastic layer 432 into the drainage system 430.

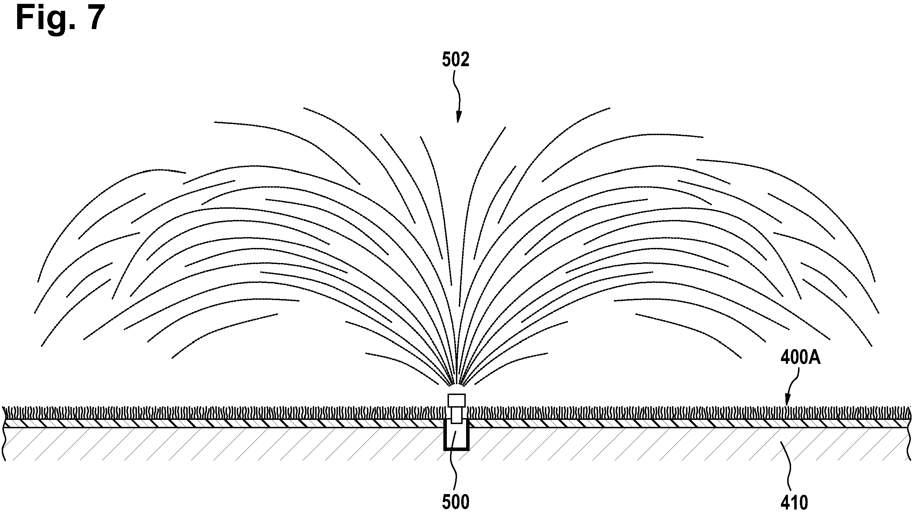

**Figure 7** shows a further example of the artificial turf e.g. 400A. In this example an automatic sprinkler system 500 has been integrated into the artificial turf 400A. The sprinkler 500 is depicted as spraying water 502 on an upper surface of the artificial turf 400A. The use of the sprinkler may be beneficial in combination with the artificial turf as it may provide an integrated watering system for an optimal watering of the artificial turf.

### List of reference numerals

- 10-23: method steps
- 31-40: method steps
- 101-103: method steps
- 201-211: method steps
- 301: crushing unit
- 302: grinding unit
- 303: grizzly
- 305: jaw crusher
- 307: Nordberg screen
- 309: cone crusher
- 311: dryer
- 313: impact crusher
- 315: Midwestern screens
- 320: sack
- 400A: artificial turf
- 402: artificial turf carpet
- 403: pile
- 404: backing
- 406: artificial grass fibers
- 408: secured to backing
- 410: ground
- 412: artificial turf infill
- 414: composite infill component
- 400B: artificial turf
- 420: sand layer
- 400C: artificial turf infill
- 432: elastic layer
- 430: drainage system
- 500: sprinkler system
- 502: spraying water.

## Claims

1. A method of disinfecting an artificial turf structure (400A-C), comprising: applying to the artificial turf structure (400A-C) a mixture of microporous zeolite mineral and at least one of copper and silver.

2. The method of claim 1, wherein the applying of the mixture comprises:
- applying the microporous zeolite mineral to the artificial turf structure;
- distributing of the copper and/or silver on the applied microporous zeolite mineral;
- exposing the mixture to water, thereby obtaining a copper and/or silver loaded zeolite.

3. The method of claim 1 or 2, the zeolite mineral having a size in the range [0.42 mm, 1.39 mm], or [0.59 mm, 1.39 mm], or [0.84 mm, 1.68 mm], or [0.18mm, 0.25mm].

4. The method of claim 1 or 3, wherein the mixture comprises a copper and/or silver loaded zeolite that is obtained before being applied to the artificial turf structure.

5. The method of claim 4, wherein the obtaining of the mixture comprises:
- adding the copper and/or the silver to water, thereby obtaining a metal solution;
- mixing the metal solution with the microporous zeolite mineral, resulting in a mixture;
- drying the resulting mixture.

6. The method of any of the preceding claims 4-5, wherein the mixing is performed such that the microporous zeolite mineral adsorbs the meta solution in an amount of at least 40 to 50% of the metal solution.

7. The method of any of the preceding claims 4-6, wherein the metal solution and the microporous zeolite mineral are exposed to a predefined pressure.

8. The method of any of preceding claim 7, further comprising introducing the metal solution and the microporous zeolite mineral in an autoclave at the predefined pressure and.

9. The method of any of preceding claims 2-8, wherein the water is a demineralized water.

10. The method of any of preceding claims 2-8, wherein the water is obtained from one of the rain and a sprinkler system.

11. The method any of preceding claims 4-10, wherein the mixture is applied to the artificial turf structure in amount of 25g/m² to 2500g/m².

12. The method of any of preceding claims, wherein the porosity of the zeolite mineral is about 10 % to about 40%, preferably from about 10% to about 35%.

13. The method of any one of claims, wherein the specific surface area of the microporous zeolite mineral is between 25 m²/g and 40 m²/g.

14. The method of any of preceding claims, wherein the microporous zeolite mineral has a selected grain size smaller than 1.5mm and a porosity of about 15% to about 20%.

15. The method of any of preceding claims, wherein the grain size distribution of said microporous zeolite mineral is as follows: 70-90% of the grains have a size in the range of about 0.4mm to about 1.5mm and about 10% to about 30% of the grains have a grain size smaller than about 0.4mm.

16. The method of any of the preceding claims, further comprising redistributing the copper and/or silver on the applied microporous zeolite mineral after a predefined time period and exposing the mixture of zeolite mineral and copper and/or silver to water.

17. A method for manufacturing an artificial turf with a disinfection capability, the method comprising:
- providing an artificial turf structure;
- applying the microporous zeolite mineral to the artificial turf structure;
- distributing of the copper and/or silver on the applied microporous zeolite mineral;
- exposing the mixture to water.

18. The method of claim 17, the zeolite mineral having a granularity in the range [0.42 mm, 1.39 mm], or [0.59 mm, 1.39 mm], or [0.84 mm, 1.68 mm], or [0.18mm, 0.25mm].

19. An artificial turf infill material comprising a copper and/or silver loaded zeolite.

20. The infill material of claim 19, wherein the copper and/or silver loaded zeolite is the mixture of any of the preceding claims 1-16.

21. The infill material of claim 19 or 20, the zeolite having a selected gain size smaller than 1.5 mm and a porosity between 15% and 20%.

22. The infill material of claim 19 or 20, wherein the zeolite has a grain size distribution as follows: 70% to 90% of the grains have a size in the range [0.4 mm, 1.5 mm] and 10% to 30% of the grains have a size smaller than 0.4 mm.

23. The infill material of claim 19 or 20, wherein 0.6% of the zeolite at most is not retainable on a 100 mesh screen.

24. The infill material of any of the preceding claims 19-23, the microporous zeolite mineral having a hardness between smaller than 3 or smaller than 4 on the Mohs scale.

25. The infill material of any of the preceding claims 19-23, wherein the moisture level in the mineral is smaller than 6%.

26. An artificial turf comprising an artificial turf carpet with a pile and artificial turf infill, wherein the artificial turf carpet comprises a backing; wherein the artificial turf carpet further comprises artificial grass fibers, wherein the artificial grass fibers are tufted into the backing, wherein the artificial grass fibers form the pile, wherein the artificial grass fibers are secured to the backing, wherein the artificial turf infill comprises a copper and/or silver loaded zeolite.

27. The artificial turf of claim 26, wherein the artificial turf further comprises a sprinkler system.
